# FASCICULE DE BREVET EUROPEEN

(11) **EP 0 446 143 B1**
(45) Date de publication et mention de la délivrance du brevet: **04.01.1995**
(21) Numéro de dépôt: 91400648.1
(22) Date de dépôt: 08.03.1991
(51) Int. Cl.: C08B 37/00, C12P 21/00, A61K 51/06

(54) **Composé polysaccharidique délipidé, procédé de préparation, compositions en comprenant**
Delipidiertes Polysaccharid, Verfahren zur Herstellung und Zusammensetungen welche dieses enthalten
Polysaccharidic delipidated compounds, process for their preparation and compositions containing them

(30) Priorité: 08.03.1990 FR 9002957
(43) Date de publication de la demande: 11.09.1991
(73) Titulaire: PIERRE FABRE MEDICAMENT, 92100 Boulogne (FR)
(72) Inventeur: Binz Hans, Lotissement Les Crêtes,, F-74160 Beaumont (FR); Dussourd D'Hinterland, Lucien, F-81100 Castres (FR); Normier, Gérard, F-81100 Castres (FR); Le Pape, Alain, F-37130 Langeais (FR); Favaron, Michel, F-74160 St Julien en Genevois (FR); Delassan, Souhail, F-74240 Gaillard (FR)
(74) Mandataire: Ahner, Francis

(56) Documents cités:
- EP-A- 0 408 444
- ACTA PATH. MICROBIOL. SCAND., SECTION C vol. 93, 1985, pages 233 - 243; G. NORMIER ET AL.: 'NK-CELL STIMULATING PROPERTIES OF A MEMBRANE PROTEOGLYCANE FROM NON-CAPSULATED KLEBSIELLA PNEUMONIAE BIOTYPE A'
- CHEMICAL ABSTRACTS, vol. 108, no. 7, 15 Février 1988, Columbus, Ohio, US; abstract no. 48726S, SINILOVA N. G. ET AL.: 'CHEMICAL STRUCTURE AND THE IMMUNOSTIMULATING ACTIVITY OF HIGH-MOLECULAR-WEIGHT POLYSACCHARIDES' page 17 ;

## Description

La présente invention concerne de nouveaux dérivés du composé dénommé D 25. Il s'agit précisément de dérivés délipidés du composé D 25. La présente invention concerne également un procédé de préparation de ces nouveaux dérivés.

La présente invention concerne également des compositions comportant ledit dérivé destinées à l'imagerie, au diagnostic ou à la thérapie notamment des foyers infectieux, inflammatoires et tumoraux, via le ciblage des macrophages. Il s'agit de compositions injectables administrables par voie lymphatique, notamment sous cutanée ou par voie intradermique ou intrapleurale, ou encore par voie i.v. respectivement, mais aussi des compositions aérosols.

La présente invention concerne également des kits de diagnostic permettant de reconstituer des compositions selon l'invention dans lesquelles le dérivé D 25 délipidé est marqué par un élément détectable.

Le produit dénommé D 25 est un composé polysaccharidique extrait de protéoglycane membranaire bactérien. Le composé polysaccharidique a été à l'origine décrit dans la demande de brevet n° EP-A-179 679 et n° EP-A-246 149.

Ce composé polysaccharidique D 25 est préférentiellement isolé d'une souche mutante non capsulée et non pathogène de Klebsiella pneumoniae biotype A, déposé à la Collection Nationale de l'Institut Pasteur sous le numéro 145-I-IP.

Dans les demandes de brevet n° EP-A-179 679 et n° EP-A-246 149, le D 25 et ses dérivés étaient présentés comme possédant des propriétés immunostimulantes, notamment vis-à-vis de l'induction d'interféron endogène et de l'activation des cellules NK (Natural Killers).

Dans ces demandes de brevet, le D 25 décrit comporte d'une part une chaîne polysaccharidique linéaire constituée par répétition environ 5 fois d'un motif monomérique de 10 sucres, et d'autre part une séquence de liaison unique, plus complexe à laquelle sont liées de courtes chaînes peptidiques.

L'unité monomérique répétitive de la chaîne polysaccharidique linéaire ne renferme que du galactose sous forme pyrane et furane dans les proportions suivantes :
3 β Gal p, 3 α Gal p, 2 β Gal f, 2 α Gal f.

La séquence de cette unité monomérique est la suivante : 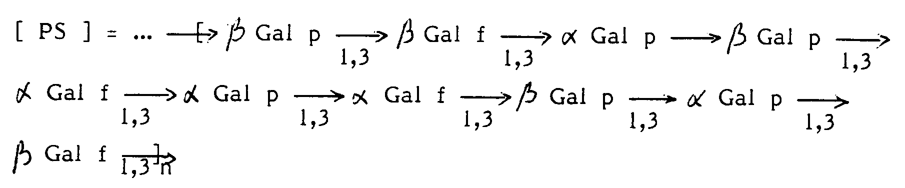
structure de liaison. La structure unique de liaison est fixée à l'extrémité de la chaîne polysaccharidique linéaire. Elle contient des résidus glucose, galactose, glucosamine, heptose et acide mannodéoxyoctulosonique. Deux courtes chaînes peptidiques sont liées à cette structure.

La séquence présumée est la suivante : 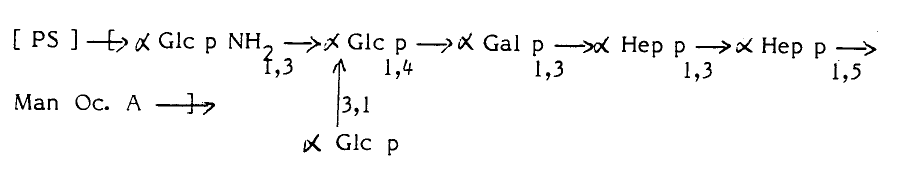
Les aminoacides suivants composant les deux chaînes peptidiques associées sont en moyenne en nombre suivant :
Acide Aspartique : 3
Acide glutamique : 2
Sérine : 1
Proline : 1
Glycine : 1,5
Alanine : 2
Valine : 1
Leucine : 1
Lysine : 1

### Abréviations :

- β Gal p: = β Galactopyranose
- α Gal p: = α Galactopyranose
- β Gal f: = β Galactofuranose
- α Gal f: = α Galactofuranose
- α Glc p: = α Glucopyranose
- α Glc p, NH₂: = α Glucosamine
- α Hep p: = α Heptose (D. Mannoheptose)
- Man Oc. A: = 3-deoxy-D-manno-octulosonic acid

Des dérivés du D 25 de type amide, ester ou éther ainsi que les sels et dérivés d'ammonium quaternaire qui sont des dérivés d'hémisynthèse du D 25, ont été décrits dans les demandes de brevet n° EP-A-246 149 et n° EP-A-288 382 au nom de la demanderesse.

Des dérivés oxydés du D 25 dans lequel le résidu galactofuranose (Gal f) de la chaîne polysaccharidique linéaire du D 25 a été transformé en arabinose, ont également été décrits dans la demande de brevet n° EP-A-288 382.

L'article "Acta Path Microbiol. Immuno. Scand. Section C, vol.93 (1985) pages 233-234, décrit un "protéoglycane membranaire de Klebsellia Pneumonia, et non pas le composé D25. Les conditions d'hydrolyse utilisées pour son obtention permettent d'extraire les lipides constitutifs de la membrane bactérienne, essentiellement composés de phospholipides et de lipoprotéines, et non des lipides associés au protéoglycane".

Le composé D 25 et ses dérives présentent une affinité pour les macrophages et constituent un agent vecteur idéal pour reconnaître et se fixer sur les foyers inflammatoires et tumoraux mobilisant des macrophages dans leur proche environnement. Telles que préparées avant la présente invention, seules des compositions aérosol pouvaient être obtenues pour l'imagerie médicale. La mise en oeuvre du D 25 radiomarqué et administré par aérosol permet l'imagerie de ganglions inflammatoires et tumoraux chez l'homme notamment dans des métastases ganglionnaires de cancer bronchique ou des métastases ganglionnaires de mélanome malin. Les essais poursuivis ont en effet démontré que toute autre voie d'administration du D 25 tel que préparé dans les demandes de brevet antérieur que la voie aérosol ne conduisait à aucun résultat satisfaisant pour l'imagerie.

Tel qu'il est décrit dans les demandes de brevet antérieures au nom de la demanderesse, le procédé de fabrication du D 25 donne en fait un produit sur lequel sont greffés par des liaisons N-glycosidiques deux acides β-hydroxymyristiques représentant environ 1,5 % de la masse du D 25, auxquels sont associées sous forme estérifiée ou sous forme libre des traces d'acides gras en C₁₆ (palmitique) représentant environ 0,5 % de la masse du D 25.

C'est la présence de ces acides gras qui confère à la molécule le caractère amphiphile qui lui permet par voie aérosol de franchir la barrière pulmonaire en fusionnant avec le surfactant alvéolaire sans nécessiter la présence de liposomes ou sans nécessiter d'avoir recours à d'autres formes galéniques. Les acides gras libres en C₁₆ remplissent le rôle d'excipient lipophile. La présence d'un pôle hydrophobe sur la molécule joue également un rôle essentiel dans l'interaction avec la membrane des monocytes et des macrophages pour permettre l'accès au récepteur du D 25 et sa fixation sur ces cellules.

Cependant, on a découvert que le caractère fortement lipophile d'une extrémité de la molécule, entraîne par les interactions hydrophobes auxquelles elle est soumise en milieu aqueux, la formation de "polymères" d'un poids moléculaire apparent supérieur à 150 KD. Ces formes "polymériques" sont en équilibre dans les solutions de D 25 avec la forme monomérique de 30 KD et peuvent représenter jusqu'à 30 % en poids. On entend ici par "polymère", plutôt des associations du type micelle ou des agrégats.

Par voie aérosol, ces "polymères" sont dissociés dans le surfactant alvéolaire et libèrent le monomère qui franchit alors la barrière pulmonaire. En revanche, par voie intraveineuse ces polymères sont très rapidement captés par le système réticulo-endothélial du foie et de la rate où ils restent fixés. C'est ce qui explique l'échec des essais qui avaient été poursuivis à l'aide de compositions injectables par voie i.v. jusqu'à présent. La voie aérosol, bien qu'elle permette la mise en évidence de foyers pathologiques de taille millimétrique, a cependant l'inconvénient de rendre impossible l'exploration de certaines régions anatomiques qui se trouvent contaminées soit directement par l'administration du radio-aérosol (massif facial) soit par la présence inévitable du produit dans les voies digestives par suite de la déglutition ou de la clairance mucocilliaire pulmonaire. Par ailleurs, l'administration d'un radio-aérosol pose des problèmes pour l'utilisation à grande échelle d'une telle forme diagnostique compte tenu des précautions qui doivent être prises pour éviter le risque de résultats faussement positifs liés à des contaminations cutanées par les patients eux-mêmes.

En ce qui concerne les compositions injectables par voie lymphatique, les techniques actuelles d'imagerie du système lymphatique mettent en oeuvre généralement, pour la radiographie, l'injection d'agents de contraste par voie sous cutanée ou intra-lymphatique et, pour la lymphoscintigraphie, l'injection sous cutanée de colloïdes ou de microparticules radiomarquées qui sont pris en charge par le flux lymphatique puis concentrés par un phénomène de filtration dans les ganglions dont ils permettent la visualisation.

La lymphoscintigraphie permet l'étude qualitative et quantitative du drainage lymphatique (traumatologie) et l'imagerie des ganglions en pathologie inflammatoire infectieuse et tumorale. Dans les deux derniers cas, les limites de la technique résident dans la réduction de la porosité pour le flux lymphatique des ganglions envahis. De ce fait, une concentration passive par tamisage des produits radiomarqués est obtenue dans le premier ganglion pathologique qui est visualisé, mais l'imagerie des étapes supérieures devient, de ce fait, impossible. Comme de telles structures pathologiques conservent cependant une certaine perméabilité au flux lymphatique, une nouvelle approche de l'imagerie par des molécules marquées est envisageable si celles-ci sont solubles et largement diffusibles. Ceci a conduit certains auteurs, ces dernières années, à utiliser de la sérum albumine radiomarquée pour la visualisation anatomique des voies lymphatiques ou d'anticorps monoclonaux dirigés spécifiquement contre des lymphocytes ou des cellules tumorales. Dans ce dernier cas, on retrouve cependant les limites bien connues de l'immunoscintigraphie liées à la faible clairance des composés mis en jeu qui hypothèque l'imagerie de foyers pathologiques de taille submillimétrique.

L'existence d'une population de macrophages présente dans les ganglions normaux et fortement accrue lors de l'inflammation ou de prolifération tumorale a conduit à mettre en oeuvre comme agent de ciblage pour ce type cellulaire le D 25 par administration locorégionale permettant de ce fait une sensibilité maximale de la détection.

La mise en oeuvre du D 25 pourrait pallier aux inconvénients des composés macromoléculaires précités. Toutefois, lorsqu'il se présente sous forme "polymérique", on retrouve l'inconvénient des agent colloïdes ou nanoparticulaires avec blocage au niveau des premiers ganglions et impossibilité d'accéder à toute la chaîne lymphatique.

Le but de la présente invention est de fournir des compositions à base de D 25 injectables que ce soit par voie i.v. ou par voie lymphatique destinées à l'imagerie, au diagnostic ou à la thérapie notamment des foyers infectieux, inflammatoires et tumoraux, via le ciblage des macrophages.

Pour ce faire, la présente invention fournit donc un nouveau dérivé délipidé du D 25, de manière à réduire le taux de polymérisation du D 25 en solution aqueuse par le biais de la réduction du caractère hydrophobe du D 25 sans l'éliminer complètement toutefois, ce qui détruirait les propriétés du produit quant à son activité comme sa fixation spécifique sur les macrophages.

Le procédé de préparation du D 25 tel qu'il est décrit dans les demandes de brevet antérieur, est caractérisé en ce que, à partir d'une souche de bactérie gram négatif, telle que Klebsiella pneumoniae, on extrait des membranes les protéoglycanes solubles dans l'eau. Les composés polysaccharidiques sont isolés après élimination des protéines présentes. Le protéoglycane soluble est préparé par solubilisation du protéoglycane membranaire brut par hydrolyse alcaline. Le protéoglycane membranaire brut est obtenu par centrifugation à partir d'un broyat cellulaire.

L'étude des conditions d'hydrolyse a permis de fixer les limites à ne pas dépasser pour conserver les propriétés du D 25, à savoir avec de la soude une molarité comprise entre 0,3 et 1 M, de préférence 0,5 à 0,75 M, le traitement étant poursuivi de préférence sous agitation à une température inférieure à 90°C, de préférence comprise entre 50 et 60°C, par exemple 56°C, le traitement étant poursuivi par exemple 1 heure. Afin d'éliminer l'excès de réactifs, après refroidissement, on neutralise la suspension par un acide, par exemple l'acide chlorhydrique. Des protéines de poids moléculaire proche de celui du composé polysaccharidique sont éliminées par hydrolyse enzymatique desdites protéines, en particulier par action de protéinase. Enfin, on sépare le composé polysaccharidique D 25 par centrifugation après son insolubilisation par précipitation.

On a découvert selon la présente invention que le rôle de l'hydrolyse alcaline n'est pas seulement de solubiliser la fraction membranaire brute bactérienne, notamment de Klebsiella pneumoniae, mais aussi de désestérifier au moins en partie des acides gras palmitiques en C₁₆ qui sont estérifiés sur des acides β-hydroxymyristiques, liés à la chaîne polysaccharidique. Mais ces acides gras libres en C₁₆ restent quand même associés au D 25 bien que non liés s'ils ne sont pas extraits.

On a découvert selon la présente invention que l'on pouvait en désestérifiant et en éliminant par extraction les acides gras palmitiques en C₁₆, obtenir un produit moins hydrophobe ne conduisant pratiquement plus à la formation de micelles ou de polymères en solution aqueuse, et même ne donnant quasiment que la forme monomérique si on le souhaite.

L'hydrophobicité du composé n'est plus alors dû essentiellement qu'aux acides β-hydroxymyristique qui sont suffisants pour conférer les propriétés d'activité et de ciblage du composé.

S'il n'est pas procédé à une hydrolyse alcaline suffisamment poussées, des acides gras en C₁₆ restent estérifiés sur les acides β-hydroxymyristiques pour partie. De même, s'il n'est pas procédé à une extraction exhaustive des acides gras libres après hydrolyse alcaline, ceux-ci se reassocient sous forme estérifiée ou restent associes de façon non lié au D 25 et provoquent la formation de micelles ou de polymères. C'est pourquoi le D 25, tel qu'il était décrit dans les demandes de brevet antérieures, comportait de 20 à 30 % en poids de forme polymérique en solution aqueuse, dans la mesure où l'on n'effectuait pas d'extraction de ces acides gras.

La présente invention a donc pour objet un composé D 25 partiellement délipidé, sous forme monomérique ou dont le taux de "polymère" en solution aqueuse ne dépasse pas 15 %, de préférence 10 % en poids. En d'autres termes, au moins 85 % en poids, de préférence 90 % du composé se présente en solution aqueuse sous forme monomérique.

En contrôlant ainsi la formation des "polymères", et donc en modulant le caractère hydrophobe de la molécule, les administrations par voie intraveineuse et par voie lymphatique, sont rendues possibles.

Ainsi, il apparaît qu'afin d'éviter la fixation hépatosplénique du D 25 administré par voie intraveineuse, il est capital de ne pas dépasser 15 % de forme polymère et de préférence de stabiliser la forme monomérique de 30 KD. En ce qui concerne la lymphoscintigraphie, la présence d'une certaine proportion de polymère (environ 10 % et jusqu'à 15 %) n'est pas un inconvénient car elle retarde l'épuration du produit et permet de visualiser les ganglions non fixants (ganglions sains), tandis que le composé reste fixé dans les ganglions pathologiques.

Enfin, l'utilisation d'un D 25 délipidé se présentant avec un taux de "polymérisation" réduit en solution aqueuse est également un avantage pour l'administration par voie aérosol, l'imagerie pouvant se faire plus rapidement.

Dans un mode préféré de réalisation de l'invention, le composé D 25 selon l'invention est extrait du protéoglycane membranaire de la bactérie Klebsiella pneumoniae ayant un poids moléculaire de 30 KD sous forme monomérique.

En particulier, la présente invention a pour objet un composé comportant une chaîne polysaccharidique linéaire composée par la répétition d'une séquence ne renfermant que des galactoses et à l'extrémité de laquelle se trouve une structure unique de liaison comportant des résidus glucoses, galactose, glucosamine, heptose et acide mannodésoxy octulosonique, sur laquelle structure deux courtes chaînes peptidiques se trouvent liées, et à l'extrémité de laquelle structure sont greffés par des liaisons N-glycosidique, respectivement sur deux glucosamines de ladite structure, deux acides β-hydroxymyristique représentant environ 1,5 % en poids du composé.

Dans les composés selon l'invention, de préférence la teneur en acides gras palmitiques qui leur sont liés sous forme estérifiée ne dépasse pas 0,01 % en poids du composé et la teneur en acides gras palmitiques qui leur sont associés sous forme libre ne dépasse pas 0,1 % en poids du composé. Dans la présente demande on entend par D 25 délipidé, un D 25 partiellement délipidé dans la mesure où les acides gras en B-hydroxymyristiques restent liés d'une part et des faibles teneurs en acides gras en C₁₆ subsistent comme mentionné ci-dessus.

La présente invention fournit également un procédé de préparation de ce dérivé de D 25 délipidé caractérisé en ce que :
a) à partir de lysats bactériens d'une souche bactérienne gram négatif on extrait le protéoglycane membranaire brut,
b) on solubilise le protéoglycane brut de l'étape a) par hydrolyse alcaline, et on récupère le protéoglycane soluble qui se trouve dans la solution aqueuse,
c) on isole un composé polysaccharidique et si nécessaire on élimine les protéines présentes dans la fraction isolée,
d) on extrait les acides gras libres associés audit compose polysaccharidique à l'aide d'un solvant organique approprié.

Parmi les bactéries gram négatif susceptibles d'être mises en oeuvre, on peut citer Klebsiella pneumoniae, Serratia marcescens et Escherichia coli ; en particulier, comme on l'a vu, Klebsiella pneumoniae qui fait l'objet d'un dépôt à la Collection Nationale de Cultures de Microorganismes (CNCM) sous le n° 145-I-IP.

Dans un mode de réalisation particulier du procédé selon l'invention, on effectue les étapes suivantes :
a) à partir d'une souche bactérienne gram négatif, on extrait le protéoglycane membranaire brut. Ce protéoglycane brut est recueilli dans le surnageant obtenu après centrifugation des lysats bactériens.
b) on solubilise le protéoglycane brut obtenu à l'étape a) par hydrolyse alcaline, en particulier avec une hydroxyl alcalin, de préférence la soude à molarité comprise entre 0,3 et 1 M, de préférence de 0,5 à 0,75 M à une température inférieure à 90°C, de préférence entre 50 et 60°C, par exemple 56°C, de préférence sous agitation, le traitement étant poursuivi pendant au moins 1 heure.
c) après refroidissement, on neutralise la suspension par un acide, par exemple l'acide chlorhydrique, et si nécessaire, on effectue une hydrolyse enzymatique des protéines présentes dans la solution, en particulier par action de protéinase.
d) le composé polysaccharidique D 25 est ensuite purifié par précipitation alcoolique et recueilli par exemple par centrifugation.
e) les acides gras libres sont extraits à l'aide d'un solvant organique approprié par exemple du chloroforme ou un mélange de chloroforme et de méthanol, par exemple le précipité du composé polysaccharidique D 25 est dispersé dans le solvant organique sous agitation puis le solvant est éliminé, puis enfin le précipité est rincé par le même solvant.
f) le précipité est repris dans l'eau, et la solution de D 25 délipidé selon l'invention est clarifiée par centrifugation, puis le surnageant est recueilli, et enfin
g) le composé D 25 délipidé selon l'invention est isolé par un procédé de fractionnement ou d'ultrafiltration conventionnel.

Notamment, dans l'étape g) on effectuera une dialyse avec de l'eau distillée par ultrafiltration sur membrane coupant à 10.000 Daltons.

Dans un mode de réalisation du procédé de l'invention utile pour obtenir notamment un très faible taux de "polymérisation", notamment inférieur à 2 %. Après l'étape f) on effectue une nouvelle hydrolyse alcaline, puis après refroidissement on neutralise la suspension par un acide par exemple l'acide chlorhydrique et éventuellement on ajoute à la solution du désoxycholate de sodium dans une proportion ne dépassant par 0,5 %, puis on précipite le composé polysaccharidique, et le précipité est repris dans l'eau, puis le composé D 25 délipidé obtenu est isolé par fractionnement ou ultrafiltration.

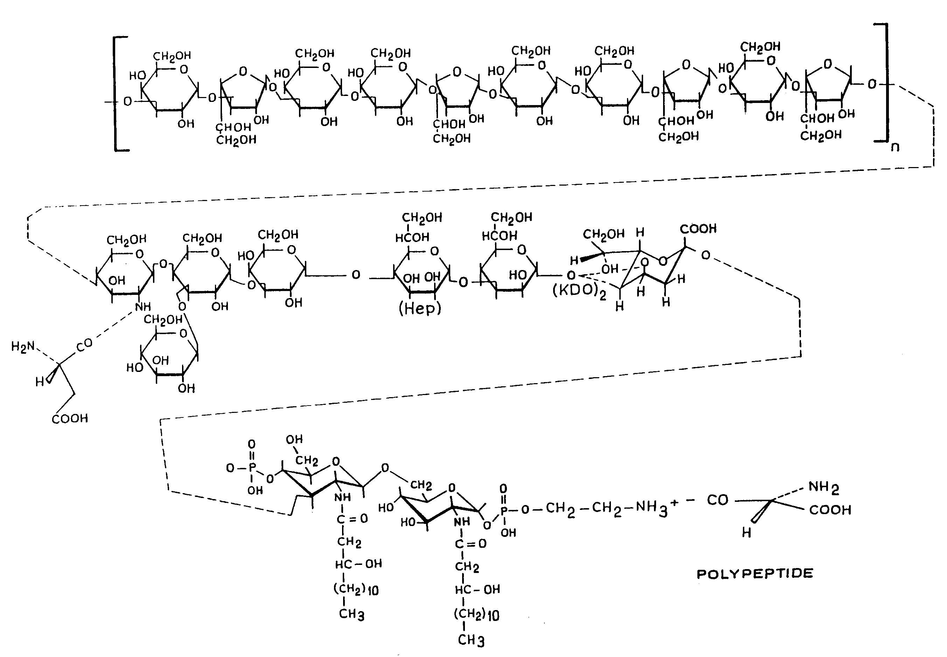

La structure monomérique de 30 KD environ du D 25 délipidé extrait de la bactérie Klebsiella pneumoniae selon l'invention est représentée sur la figure 1.

La structure unique de liaison fixée à l'extrémité de la chaîne polysaccharidique linéaire contient outre la séquence indiquée précédemment, deux glucosamines terminales sur chacune desquelles est lié un acide β-hydroxymyristique par une une liaison N-glycosidique. Une autre chaîne peptidique est liée à la dernière glucosamine par l'intermédiaire d'un groupe éthyl aminophosphate. Les courtes chaînes peptidiques se terminent par un acide aspartique. Une chaîne peptidique est liée à la première glucosamine adjacente à l'unité monomérique du PS.

Enfin, la présente invention a pour objet des compositions destinées à l'imagerie, au diagnostic ou à la thérapie caractérisées en ce qu'elles comportent le composé D 25 partiellement délipidé selon l'invention.

Dans un mode préférentiel de réalisation de l'invention, les compositions sont destinées à l'imagerie et au diagnostic ou la thérapie des foyers infectieux inflammatoires et tumoraux via le ciblage des macrophages.

Les compositions selon l'invention peuvent être respectivement injectables par voie i.v. par voie lymphatique notamment sous cutanée ou intrapleurale ou administrables par voie aérosol.

Lorsque les compositions sont destinées à l'imagerie et au diagnostic, le composé polysaccharidique selon l'invention peut être marqué par un élément détectable tel qu'un élément radioactif paramagnétique ou fluorescent.

Ledit composé polysaccharidique peut également être détectable indirectement, c'est-à-dire qu'il peut être détecté via une substance elle-même marquée par un élément détectable tel que mentionné précédemment, ladite substance reconnaissant et se liant spécifiquement audit composé polysaccharidique (D 25 délipidé). A titre de substance reconnaissant et se liant spécifiquement audit composé polysaccharidique D 25 délipidé, on peut citer un anticorps dressé contre ledit composé.

Les kits d'imagerie ou de diagnostic in vivo ou ex vivo selon l'invention, comporteront donc soit le composé polysaccharidique D 25 délipidé et des moyens pour le marquer, soit le composé polysaccharidique D 25 délipidé et une substance reconnaissant ledit composé et des moyens pour marquer ladite substance.

Un champ d'application du dérivé D 25 décrit selon la présente invention est l'imagerie scintigraphique ou la détection per opératoire lorsque cet agent est couplé à un isotope radioactif. Ces radionucléides peuvent en effet être détectables par visualisation ou par comptage per opératoire par une sonde guidée manuellement. Il s'agit alors d'un diagnostic par comptage et non d'imagerie. On peut citer comme élément radioactif approprié selon l'invention, un radionucléide détectable par scintigraphie tel que le technécium Tc^{99m} ou encore à titre non limitatif, l'iode ¹²³ et l'indium ¹¹¹.

Le dérivé D 25 délipidé peut être également associé à un agent paramagnétique tel que le gadolinium par exemple pour constituer un produit de contraste en imagerie par résonnance magnétique (IRM).

En plus des possibilités d'imagerie des foyers infectieux, inflammatoires ou tumoraux, le D 25 délipidé constitue un marqueur d'organe pour le foie et la rate normaux ou pathologiques.

Plus précisément, les compositions injectables comportant du D 25 délipidé avec un taux de polymérisation inférieur à 15 %, de préférence de 5 à 15 % sont particulièrement appropriées pour l'imagerie, le diagnostic et la thérapie des foyers infectieux, inflammatoires et tumoraux des voies lymphatiques via le ciblage des macrophages.

Un nouvelle application lymphoscintigraphique du composé selon l'invention concerne l'imagerie des ganglions médiastinaux.

De même, les compositions injectables comportant du D 25 délipidé avec un taux de polymérisation inférieur à 5 % sont les plus appropriés pour l'imagerie, le diagnostic et la thérapie des foyers infectieux, inflammatoires et tumoraux par voie i.v. via le ciblage des macrophages.

Enfin, compte tenu de ses caractéristiques permettant le ciblage des macrophages, le composé selon l'invention peut être bien entendu utilisé à des fins thérapeutiques comme agent de vectorisation pour la radiothérapie ciblée, pour la vectorisation de substances cyto toxiques ou encore pour la vectorisation d'immunogène et/ou d'agents immunomodulateurs.

Enfin, le D 25 délipidé selon l'invention de par ses propriétés immunopharmacologiques intrinsèques (activateur des macrophages, inducteur d'interféron α, activateur de cellules NK, inducteur de la production d'interleukine 1, etc) pourra être également utilisé directement à titre de médicament notamment comme agent immunostimulant vis-à-vis des métastases et d'infections notamment intracellulaires.

La présente invention a également pour objet des dérivés du D 25 délipidé, il s'agit des dérivés oxydés, les dérivés oxydés de D 25 s'entendant de composés dans lesquels les résidus galactofuranose (Gal f) de la chaîne polysaccharidique linéaire ont été transformés en arabinose ; et les dérivés de type amide, ester ou éther, ainsi que leur sels et dérivés d'ammonium quaternaires.

D'autres caractéristiques et avantages de la présente invention apparaîtront à la lumière des exemples qui vont suivre.

La figure 1 représente la structure du D 25 délipidée.

### EXEMPLE 1 : PROCEDES DE FABRICATIONS (donnés à partir de 1 kg de biomasse de K. pneumoniae exprimé en poids sec)

### 1. Obtention de lysats bactériens clarifiés

La biomasse de Klebsiella pneumoniae est obtenue par culture en fermenteur en milieu liquide dans des conditions classiques. La seule contrainte spécifique étant l'arrêt brusque de la croissance par refroidissement à + 4°C en fin de phase exponentielle de croissance pour préserver l'intégrité des structures biologiques.

La biomasse est séparée du milieu de culture par centrifugation continue à froid sur séparateur industriel de type Sharples ou Westfalia. Après lavage par remise en suspension dans du sérum physiologique stérile et centrifugation, le concentrat cellulaire est stocké congelé en attendant d'être traité.

Le concentrat bactérien est décongelé dans un réacteur et mis en suspension dans du tampon tris-HCl (10 mM) pH 7,0 contenant MgCl₂ (10 mM) et NaCl (0,15 mM) à 4°C pour avoir une concentration finale équivalente à 50 g de cellules sèches par litre de suspension. On ajoute ensuite 5 mg de DNase par litre de suspension.

Les cellules microbiennes sont ensuite désintégrées par passage en continu sur des broyeurs industriels de type APV Manton Gaulin. Le lysat bactérien ainsi obtenu est soumis à une première clarification continue à 15.000 xg sur séparateur Sharples à 4°C pour éliminer les résidus de broyage et les germes non broyés. Le culot de centrifugation est éliminé et le surnageant recueilli, il constitue le lysat clarifié.

### 2. Obtention du protéoglycane membranaire

Le lysat bactérien clarifié est acidifié à pH 4,2 ± 0,2 par l'acide acétique et laissé 30 minutes au repos à + 4°C. Le précipité d'impuretés formé est éliminé par centrifugation continue à 15.000 xg sur Sharples. Le surnageant opalescent contenant le protéoglycane membranaire est neutralisé par NaOH, puis dialysé par ultrafiltration à volume constant contre de l'eau distillée sur membrane coupant à 10.000 Daltons. Lorsque la resistivité est égale ou supérieure à 1000 Ω cm⁻¹ le volume est concentré par filtration sur la membrane à 10.000 Daltons jusqu'à 6 l pour 1 kg d'équivalent en cellules séches de biomasse initiale.

Le titre en protéines de la suspension de protéoglycane membranaire est dosé par la réaction du biuret puis ajusté par dilution avec de l'eau distillée pour avoir une concentration finale de 10 mg de protéines par ml.

Cette suspension de protéoglycane membranaire de K. pneumoniae sera utilisée ensuite pour l'extraction du D 25 en vue de ses différentes applications.

### 3. Préparation du D 25 délipidé pour utilisation en lymphoscintigraphie (environ 10 % de composé sous forme "polymère" en solution aqueuse) (code SJD252)

A la suspension de protéoglycane membranaire obtenue en 2, on ajoute de l'hydroxyde de sodium concentré jusqu'à une normalité finale de 0,5 à 0,75 N (de préférence 0,5 N). La solution est ensuite portée à 56°C et maintenue sous agitation pendant 1 heure à cette température. Après refroidissement à température ambiante, la solution est neutralisée par l'acide chlorhydrique.

A la solution neutralisée on ajoute du Tris q.s.p. M/100 et de l'EDTA q.s.p. M/1000, puis on ajuste le pH à 7,4. On procède alors à une digestion enzymatique pendant 2 heures à 37°C en présence de 0,4 g/l de protéinase K et 0,1 g/l de lysozyme.

Le D 25 est séparé par précipitation avec 2 volumes d'éthanol préalablement refroidi à 20°C. Après un repos de 30 minutes à 4°C, le précipité est recueilli par centrifugation continue sur Sharples avec un débit de 6 l/minute.

Le culot de D 25 est aussitôt dispersé dans 1 l de mélange chloroforme : méthanol (3 : 1) par passage au Turrax à température ambiante, par filtration sur verre fritté n° 3 et le précipité rincé sur le filtre par 500 ml du même mélange chloroforme : méthanol. Le précipité est séché sous courant d'azote puis repris dans 1,5 l d'eau distillée par dispersion au Turrax, et maintenu sous agitation pendant 1 nuit à + 4°C.

La solution de D 25 délipidée est clarifiée par centrifugation à 30.000 g pendant 60 minutes à + 4°C, et le surnageant est dialysé contre de l'eau distillé sur membranes coupant à 10.000 Daltons, jusqu'à atteindre une résistivité égale ou supérieure à 5000 Ω cm⁻¹. Le volume est ajusté à 1,5 l par de l'eau distillée et le pH ajusté à 7,2.

Le D 25 est ensuite stérilisé par filtration sur membrane 0,22 µm et lyophilisé stérilement. Ce lyophilisat constitue le SJD252, principe utilisé ci-après pour la lymphoscintigraphie.

### 4. Préparation du D 25 pour injection intraveineuse (code SJD253) (moins de 5 % de composé sous forme "polymère" en solution aqueuse

A la suspension de protéoglycane membranaire obtenue en 2, on ajoute de l'hydroxyde de sodium concentré pour avoir une normalité finale de 0,5 à 0,75 N (de préférence 0,75 N). La solution est ensuite portée à 56°C et maintenue sous agitation pendant 1 heure à cette température. Après refroidissement à température ambiante, la solution est neutralisée par l'acide chlorhydrique.

A la solution neutralisée on ajoute du tris q.s.p. M/100 et de l'EDTA q.s.p. M/1000, puis on ajoute le pH 7,4. On procède alors à une digestion enzymatique pendant 2 heures à 37°C en présence de 0,4 g/l de protéinase K et 0,1 g/l de lysozyme.

Le D 25 est séparé par précipitation avec 2 volumes d'éthanol prélablement refroidi à 20°C. Après un repos de 30 minutes à + 4°C, le précipité est recueilli par centrifugation continue sur Sharples avec un débit de 6 l/minute.

Le culot de D 25 est aussitôt dispersé dans 2 l de mélange chloroforme : méthanol (3 : 1) par passage au Turrax à température ambiante, avec agitation intermittente pendant 15 minutes. Le solvant est éliminé par filtration sur verre fritté n° 3 et le précipité est rincé par 1 l du même mélange chloroforme : méthanol. Le précipité est séché sous courant d'azote puis repris dans 2 l d'eau distillée par dispersion au Turrax, et maintenu sous agitation pendant 1 nuit à + 4°C.

La solution de D 25 délipidée est clarifiée par centrifugation à 30.000 xg pendant 60 minutes à 4°C, et le surnageant est recueilli puis additionné d'hydroxyde de sodium concentré pour avoir une normalité finale de 0,5 N. La solution est ensuite portée à 56°C et maintenue sous agitation pendant 1 heure à cette température. Après refroidissement à température ambiante, la solution est neutralisée par l'acide chrorhydrique.

On ajoute à cette solution du désoxycholate de sodium q.s.p. 0,5 %, puis on agite à 25°C pendant 30 minutes vigoureusement. Deux volumes d'éthanol préalablement refroidi à + 4°C sont ajoutés pour précipiter le D 25. Après 30 minutes de repos à 4°C, le précipité est recueilli par centrifugation continue sur Sharples avec un débit de 6 l/minute.

Le culot de D 25 est aussitôt repris dans 2 l d'eau distillée par dispersion au Turrax, puis maintenu sous agitation pendant 1 nuit à + 4°C. La solution est dialysée contre de l'eau distillée sur membranes coupant à 10.000 Daltons, jusqu'a atteindre une résistivité égale ou supérieure à 5000 Ω cm⁻¹. Le volume est ajusté à 1,5 l par de l'eau distillée et le pH ajusté à 7,2.

Le D 25 est ensuite stérilisé par filtration sur membrane 0,22 µm et lyophilisé stérilement.

Ce lyophilisat constitue le SJD253, principe ci-après pour la voie intraveineuse.

### Schémas d'extraction du D 25 pour les différentes applications d'imagerie scintigraphique

A partir d'une suspension de protéoglycane membranaire de Klebsiella pneumoniae ajustée à 10 mg/ml de protéines et pour 1 kg de biomasse initiale (exprimé en poids sec)

### I - D 25 : SJD251 (environ 20 à 30 % du polymère) (procédés antérieurs)

1. Hydrolyse alcaline (NaOH 0,5 N - 1 heure à 56°C)
2. Neutralisation
3. Digestion enzymatique (protéinase K 0,1 g/l + lysozyme 0,1 g/l pendant 2 heures à 37°C.
4. Précipitation alcoolique (2 volumes d'éthanol à 20°C).
5. Reprise par CH₃COONa 0,5 M.
6. Précipitation alcoolique (id. 4).
7. Reprise eau distillée.
8. Clarification (60 min. à 30.000 g).
9. Dialyse contre H₂O (≧ 2.500 Ω cm⁻¹ sur membrane 10.000 d.)
10. Stérilisation sur membrane 0,22 µm.
11. Lyophilisation stérile : SJD251.

### II - D 25 pour lymphoscintigraphie : SJD252 (environ 7-12 % de polymères)

1. Hydrolyse alcaline (NaOH 0,5 N - 1 h à 56°C)
2. Neutralisation
3. Digestion enzymatique (protéinase K 0,4 g/l + lysozyme 0,1 g/l pendant 2 heures à 37°C).
4. Précipitation alcoolique (2 volumes d'éthanol à 20°C).
5. Délipidation par chloroforme : méthanol. Séchage.
6. Reprise par l'eau distillée (1 nuit à 4°C).
7. Clarification (60 min. à 30.000 g).
8. Dialyse contre H₂O (≧ 5.000 Ω cm⁻¹ sur membrane 10.000 d.)
9. Stérilisation sur membrane 0,22 µm.
10. Lyophilisation stérile : SJD252.

### III - D 25 par voie intraveineuse : SJD253 (quasiment monomérique taux de polymère < 5 %)

1. Hydrolyse alcaline (NaOH 0,75 N - 1 heure à 56°C).
2. Neutralisation
3. Digestion enzymatique (protéinase K 0,4 g/l + lysozyme à 20°C)
4. Précipitation alcoolique (2 volumes d'éthanol à 20°C).
5. Délipidation par chloroforme : méthanol. Séchage.
6. Reprise par l'eau distillée (1 nuit à 4°C).
7. Clarification (60 min. à 30.000 g).
8. Hydrolyse alcaline (NaOH 0,5 N - 1 heure à 56°C).
9. Neutralisation.
10. Traitement par le désoxycholate de Na (0,5 % - 30 minutes à 25°C).
11. Précipitation alcoolique (2 volumes d'éthanol à 4°C).
12. Reprise par l'eau distillée (1 nuit à 4°C).
13. Dialyse contre H₂O (≧ 5.000 Ω cm⁻¹ sur membrane 10.000 d.).
14. Stérilisation sur membrane 0,22 µm.
15. Lyophilisation stérile : SJD253.

### EXEMPLE 2 : COMPOSITIONS DESTINEES A L'IMAGERIE, AU DIAGNOSTIC DES FOYERS INFECTIEUX, INFLAMMATOIRES ET TUMORAUX, VIA LE CIBLAGE DES MACROPHAGES

Le D 25 délipidé selon l'invention et ses dérivés se sont avérés présenter une affinité pour les cellules du type monocytes - macrophages qui en font plus particulièrement un agent vecteur idéal pour reconnaître et se fixer sur les foyers inflammatoires et tumoraux mobilisant ces cellules.

Des études de liaison du D 25 délipidé marqué par l'isothiocyanate de fluorescéine (D 25-FITC) ont été réalisées sur des leucocytes humains et sur différents types de cellules en lignées. A la dose de 0,1 à 1 mg/ml, le D 25-FITC incubé en présence de monocytes, de lymphocytes et de ganulocytes humains montre par cytofluorimétrie de flux une fixation élective sur la population monocytaire. Les courbes représentant l'évolution de la fixation du D 25-FITC sur ces cellules en fonction de sa concentration dans le milieu montrent l'affinité préférentielle des monocytes pour le D 25 délipidé dans la gamme de concentration 0,5-10 mg/ml avec une augmentation par un facteur 2,2 à 6,5 de l'index de fluorescence pour les monocytes (1,1 à 1,6 pour les lymphocytes et les polynucléaires).

La mise en oeuvre de lignées cellulaires de différents types : cellules hématopoïétiques (K562), promyélocytes (H160), monocytes-like (U937), lymphoblastes (IM-9), myélomateuses (U-266) et leucémiques T (MOLT-4) en étudiant la spécificité de la liaison du D 25-FITC par déplacement après compétition avec le D 25 non marqué confirme l'électivité de la fixation pour la lignée monocytaire (tableau 1).

**Tableau 1**

| Etude de la liaison du D 25 délipidé fluorescent (isothiocyonate de fluorescéine) à des lignées cellulaires d'origine humaine | | | |
|---|---|---|---|
| Type cellulaire | Intensité moyenne de fluorescence | | |
| | bruit de fond | Fluorescence initiale * | Fluorescence après compétition par D 25 délipidé non marqué** |
| Lignée monocytaire | | | |
| K562 | 2.9 | 5.7 | 3.7 (-71)*** |
| HL-60 | 2.8 | 5.7 | 4.8 (-31) |
| U-937 | 3.1 | 6.6 | 4.5 (-60) |

| Autres lignées | | | |
|---|---|---|---|
| IM-9 | 2.8 | 3.6 | 3.4 |
| U-266 | 3.5 | 4.5 | 4.1 |
| MOLT-4 | 3.6 | 3.6 | 3.3 |

| | | | |
|---|---|---|---|
| * D 25-FITC 1 mg/ml | | | |
| ** D 25-FITC 1 mg/ml après compétition par le D 25 10 mg/ml | | | |
| *** pourcentage d'inhibition de la liaison du D 25-FITC par le D 25. | | | |

### 2.1. Imagerie via le ciblage des macrophages par voie i.v.

La stratégie de ciblage in vivo des macrophages par l'agent vecteur faisant l'objet de la présente invention a été évaluée par scintigraphie chez l'animal dans deux modèles de pathologie expérimentale :
1) l'arthrite immunologique induite par l'ovalbumine chez le lapin,
2) la réaction inflammatoire associée à l'irradiation ponctuelle du muscle de la cuisse chez le porc.

### Résultats expérimentaux

1°/ Des lapins néozélandais adultes ont été sensibilisés par de l'ovalbumine associée à de l'adjuvant de Freund et administrée par injections I.D. répétées sur le dos. Après un mois de sensibilisation, une injection déclenchante d'ovalbumine est réalisée dans l'articulation correspondant au genou droit. Après 15 jours d'évolution de la pathologie et régression de l'oedème initial, les animaux sont soumis pendant trois mois à des scintigraphies par le SJD253 ou le méthylène diphosphonate (traceur ostéo-articulaire de référence) marqués par le ^{99m}Tc et administré par voie i.v.

Les examens ont été réalisés chez des animaux vigiles en utilisant une PHOGAMMACAMERA (Siemens) équipée d'un collimateur parallèle et connectée à un système informatique TIM 512 (Medimag, Besançon). Une acquisition statique de 1 minute avec une matrice de 128 x 128 pixels était réalisée une heure après injection intraveineuse des produits marqués par 1 mCi de ^{99m}TcO₄⁻ (CIS, Gif-sur-Yvette).

Le marquage du SJD253 était réalisé à partir de 500 µg de produit stérile et apyrogène réduits sous vide par 100 µg de chlorure stanneux ou d'un autre halogénure d'étain stanneux (SnF₂...) selon la technique de LIN et al., J. Nucl. Med., 1971, 12, 204-211, en mettant en oeuvre 2 mCi de ^{99m}TcO₄⁻.

Le rendement de marquage déterminé par radiochromatographie de partage en couche mince dans le solvant méthanol/eau (85/15) était supérieur à 99,5 % sans présence détectable de colloïde.

Pour les examens au méthylène diphosphonate, une trousse TCK14M (CIS, Gif-sur-Yvette) contenant 2,5 mg d'acide méthylène diphosphonique et 0,25 mg de chlorure stanneux dihydraté a été utilisée. Le marquage était réalisé extemporanément par addition de 5 mCi de ^{99m}TcO₄⁻. Le rendement de marquage, déterminé par radiochromatographie de partage en milieu méthanol/eau 85:15 était supérieur à 99,5 % sans détection appréciable de colloïde. Le quart de la préparation correspondant à une activité de 1 mCi était injecté par voie intraveineuse aux animaux une heure avant l'examen scintigraphique.

### 2°/ Expérimentation sur les foyers d'irradiation chez le porc

Des irradiations par une source de 100 Ci d'Iridium 192 ont été réalisées sur la face latérale de la cuisse droite de porcs de souche Large White en délivrant une dose d'irradiation de 60 Gy à 2 cm. L'irradiation est localisée à 10 cm en retrait du fémur et à égale distance de la crête du trochanter et de l'épichondyle latéral. Les animaux ont été opérés 15 jours après irradiation, sous anesthésie gazeuse pour réaliser une résection de la peau irradiée et éviter des réactions inflammatoires non spécifiques et une infection induite par la radionécrose. Les examens scintigraphiques ont été réalisés 60 à 70 jours après l'opération, après contrôle de l'absence de tout oedème ou de réaction inflammatoire superficielle liée au processus de cicatrisation. Les examens ont été effectués avec une gamma caméra de type GCA (Toshiba) équipée d'un collimateur parallèle et réglée sur le pic photoélectrique de 140 Kev du ^{99m}Tc avec une fenêtre de 20 %. La digitalisation et le stockage des images étaient assurés par un analyseur d'images Péricolor 1000. Pour un animal de 70 kg, la dose administrée correspondait à 1 mg de SJD253 marqué en présence de 200 µg de chlorure stanneux par 10 mCi de ^{99m}TcO₄⁻ une heure avant l'examen scintigraphique. Après contrôle de la qualité du marquage par radiochromatographie de partage en couche mince selon le protocole précédemment décrit, le D 25 marqué était injecté par voie intraveineuse dans la veine marginale de l'oreille une heure avant l'examen scintigraphique.

### 2.2. Ciblage des macrophages par voie lymphatique

Cette stratégie de ciblage des macrophages pour la lymphoscintigraphie a été évaluée chez l'animal sain et en pathologie expérimentale.

### 1) Chez le chien et le singe sains :

L'étude a été réalisée chez l'animal vigile par injection sous-cutanée dans deux espaces interdigités des pattes postérieures de 50 µl par point d'injection de solution contenant 500 µg de D 25 marqué par 500 µCi de ^{99m}Tc en présence de 100 µg d'un halogénure d'étain stanneux (par exemple SnCl₂, SnF₂ ...) selon la technique de Lin et al. J. Nucl. Med., 1971, 12, 204-211. Un examen de repérage a été réalisé avec des produits radiopharmaceutiques actuellement commercialisés pour la lymphoscintigraphie : des nano colloïdes d'albumine humaine (Nanocoll, Solco, Bâle) ou du sulfure de Rhénium colloïdal (TCK-17, CIS, Gif-sur-Yvette) marques au ^{99m}Technétium.

Les D 25 étudiés correspondent :
- au produit naturel identifié SJD251,
- au dérivé optimisé pour la lymphoscintigraphie et obtenu selon la technique décrite dans le présent brevet pour limiter les interactions hydrophobes liées au caractère amphiphile de la molécule naturelle ; le produit ainsi optimisé est identifié SJD252. Le rendement de marquage des fractions de D 25 a été déterminé par radiochromatographie de partage en couche mince dans le solvant méthanol/eau (85/15) et était supérieur à 99,5 % sans présence détectable de colloïde.

Les examens ont été réalisés sur une PHOGAMMACAMERA (SIEMENS) équipée d'un collimateur parallèle et connectée à un système informatique TIM 512 (Médimag, Besançon). Des acquisitions dynamiques ont été réalisées au niveau des membres postérieurs à raison d'une image par minute pendant 1 heure. Les résultats montrent une cinétique quasiment identique de la distribution lymphatique du SJD251, du Nanocoll et du sulfure de Rhénium colloïdal, caractérisée par une visualisation des relais ganglionnaires au bout de 30 minutes qui persiste jusqu'à une heure, voire plus longtemps.

Pour le D 25 délipidé (SJD252) optimisé pour la lymphoscintigraphie, la prise en charge par le système lymphatique est extrêmement rapide, dès les cinq premières minutes suivant l'injection et l'imagerie des relais ganglionnaires est obtenue en 10-20 minutes avec un contraste scintigraphique voisin de celui obtenu par les radiopharmaceutiques de référence. Par contre, chez l'animal sain, une clairance rapide entraînant la disparition des images ganglionnaires intervient entre 30 et 60 minutes post injection.

En pathologie expérimentale, 2 modèles ont été mis en oeuvre :
- L'infection à Corynebacterium pseudotuberculosis chez l'agneau : la dissémination bactérienne à partir du granulome infectieux situé à l'extrémité du membre postérieur s'effectue par les voies de drainage lymphatique entraînant la formation de 2 ganglions infectieux qui deviennent imperméables aux agents lymphoscintigraphiques de référence.
- Un modèle de ganglion axillaire inflammatoire réactionnel induit par injection I.D. de tuberculine chez le singe Papio-papio préalablement immunisé par le BCG.

Dans le cas des ganglions infectieux chez le mouton, le SJD252 permet l'imagerie des 2 ganglions pathologiques dans le 30-60 minutes suivant l'injection avec une clairance quasi nulle pendant au moins 90 minutes. Dans le membre contro-latéral sain, l'examen pratiqué permet l'imagerie des ganglions normaux pendant 30 minutes seulement puisque la clairance du produit non fixé intervient ensuite.

Pour le ganglion inflammatoire induit par la tuberculine chez le singe, un résultat identique est obtenu dans les mêmes délais mais l'image persiste pendant 3 heures.

Une nouvelle application lymphoscintigraphique du SJD252 concerne l'imagerie des ganglions médiastinaux. Ce développement a été réalisé chez le singe Papio-papio vigile par injection intradermique ou intrapleurale de 250 µg de produit marqué par 250 µCi de ^{99m}Technétium. La clairance pleurale de l'agent scintigraphique s'accompagne dans les 3 heures post injection d'une activité qui croît avec le temps au niveau des ganglions du médiastin postérieur et en permet ainsi l'imagerie.

Les principaux territoires habituellement explorables par cette voie sont les membres inférieurs et supérieurs et les chaînes ganglionnaires de la région mammaire après injection sous cutanée ou intra-dermique du produit dans les territoires de drainage lymphatique. Les possibilités d'exploration ont été étendues à l'imagerie des ganglions médiastinaux dont l'importance est considérable en pathologie inflammatoire et tumorale ; elle est réalisée par injection intra-pleurale du produit.

## Revendications (Revendications pour l'(les) Etat(s) contractant(s) suivant(s): DE, GB, FR, IT, NL, SE, CH, LI, BE, AT, LU, DK)

1. Composé polysaccharidique D 25 de poids moléculaire de 30 kD extrait des protéoglycanes membranaires de la bactérie Klebsiella pneumoniae, comportant une chaîne polysaccharidique linéaire composée par la répétition d'une séquence ne renfermant que des galactoses et à l'extrémité de laquelle se trouve une structure unique de liaison comportant des résidus glucoses, galactose, glucosamine, heptose et acide mannodésoxy octulosonique, sur laquelle structure deux courtes chaînes peptidiques se trouvent liées, et à l'extrémité de laquelle structure sont greffés par des liaisons N-glycosidique respectivement deux acides bêta-hydroxymyristique représentant environ 1,5 % en poids du composé, composé partiellement délipidé de sorte que lui sont associés sous forme libre ou estérifiée des acides gras palmitiques représentant moins de 0,5% en poids du composé, et qu'au moins 85 % en poids, de préférence 90 % du composé, ne se présente pas en solution aqueuse sous forme d'associations du type micelle ou d'agrégats, et ses dérivés.

2. Composé selon la revendication précédente, caractérisé en ce que la teneur en acides gras palmitiques qui lui sont liés sous forme estérifiée ne dépasse pas 0,01 % en poids du composé et la teneur en acides gras palmitiques qui lui sont associes sous forme libre ne dépasse pas 0,1 % en poids du composé.

3. Procédé de préparation d'un composé selon l'une des revendications précédentes, caractérisé en ce que :
a) à partir de lysats bactériens d'une souche bactérienne gram négatif on extrait le protéoglycane membranaire brut,
b) on solubilise le protéoglycane brut de l'étape a) par hydrolyse alcaline, avec de la soude à molarité comprise entre 0,3 et 1M, de sorte que les acides gras palmitiques estérifiés sur les acides bêta-hydroxymyristique sont désestérifiés au moins en partie, et on récupère le protéoglycane soluble qui se trouve dans la solution aqueuse,
c) on isole un composé polysaccharidique et si nécessaire on élimine les protéines présentes dans la fraction isolée,
d) on extrait les acides gras libres associés audit composé polysaccharidique à l'aide d'un solvant organique approprié.

4. Procédé selon la revendication 3, caractérisé en ce que :
a) à partir de lysats bactériens d'une souche bactérienne gram négatif, on extrait le protéoglycane membranaire brut,
b) on solubilise le protéoglycane brut obtenu de l'étape a) par hydrolyse alcaline avec la soude à molarité comprise entre 0,3 et 1 M, de préférence 0,5 à 0,75 M, à une température inférieure à 90°C, de préférence entre 50 et 60°C, le traitement étant poursuivi pendant au moins une heure,
c) après refroidissement, on neutralise la suspension par un acide, et si nécessaire on effectue une hydrolyse enzymatique des protéines présentes dans la solution,
d) un composé polysaccharidique est ensuite purifié par précipitation alcoolique et recueilli par exemple par centrifugation,
e) les acides gras libres sont extraits à l'aide d'un solvant organique approprié par exemple du chloroforme ou un mélange de chloroforme et de méthanol, en dispersant ledit composé polysaccharidique dans ledit solvant organique, puis en éliminant le solvant et en rinçant le précipité après élimination du solvant de préférence par le même solvant,
f) le précipité est repris dans l'eau et une solution de D 25 délipidée selon l'invention est clarifiée par centrifugation, puis le surnageant est recueilli, et enfin
g) un composé D 25 délipidé est isolé par un procédé de fractionnement ou d'ultrafiltration.

5. Procédé selon la revendication 4, utile pour obtenir notamment un très faible taux de polymérisation notamment inférieur à 2 %, caractérisé en ce qu'après l'étape f), on effectue une nouvelle hydrolyse alcaline ; puis après refroidissement on neutralise la suspension par un acide et éventuellement on ajoute à la solution du désoxycholate de sodium dans une proportion ne dépassant pas 0,5 % ; puis on précipite le composé polysaccharidique ; le précipité est repris dans l'eau et le composé D 25 délipidé obtenu est isolé par fractionnement ou ultrafiltration.

6. Composition destinée à l'imagerie, au diagnostic ou à la thérapie, caractérisée en ce qu'elle comporte le composé D 25 partiellement délipidé ou un de ses dérivés selon l'une des revendications précédentes.

7. Composition selon la revendication 6, destinée à l'imagerie et au diagnostic des foyers infectieux, inflammatoires et tumoraux via le ciblage des macrophages.

8. Composition selon l'une des revendications 6 et 7 caractérisée en ce qu'elle est injectable par voie i.v..

9. Composition selon l'une des revendications 6 et 7, caractérisée en ce qu'elle est injectable par voie lymphatique, notamment par voie sous cutanée ou voie intrapleurale.

10. Composition selon l'une des revendications 6 et 7, caractérisée en ce qu'elle est administrable par voie aérosol.

11. Composition d'imagerie ou de diagnostic selon l'une des revendications 6 à 10, caractérisée en ce que le composé polysaccharidique D 25 partiellement délipidé est marqué par un élément détectable tel qu'un élément radioactif, paramagnétique ou fluorescent.

12. Composition d'imagerie et de diagnostic selon l'une des revendications 6 à 10, caractérisée en ce qu'elle comporte le composé polysaccharidique selon l'invention non marqué et en ce que la composition fait partie d'un kit d'imagerie et de diagnostic comportant une deuxième composition comprenant une substance capable de reconnaître ledit composé polysaccharidique notamment un anticorps, ladite substance étant elle-même marquée par un élément détectable tel qu'un élément radioactif, paramagnétique ou fluorescent.

13. Composition selon l'une des revendications 11 et 12, caractérisée en ce que l'élément radioactif est un radionucléide détectable par scintigraphie.

14. Composition selon la revendication 13, caractérisée en ce que le radionucléide est le technétium ^{99m}, l'iode ¹²³, ou l'indium¹¹¹.

15. Utilisation du composé selon l'une des revendications 1 à 5 à des fins thérapeutiques comme agent de vectorisation pour la radiothérapie ciblée, pour la vectorisation de substances cytotoxiques ou encore pour la vectorisation de substances immunogènes et/ou d'agents immunomodulateurs.

16. Utilisation du composé selon l'une des revendications 1 à 5 à titre de médicament.

## Revendications (Revendications pour l'(les) Etat(s) contractant(s) suivant(s): ES, GR)

1. Procédé de préparation d'un composé polysaccharidique D 25 de poids moléculaire de 30 kD extrait des protéoglycanes membranaires de la bactérie Klebsiella pneumoniae, comportant une chaîne polysaccharidique linéaire composée par la répétition d'une séquence ne renfermant que des galactoses et à l'extrémité de laquelle se trouve une structure unique de liaison comportant des résidus glucoses, galactose, glucosamine, heptose et acide mannodésoxyoctulosonique, sur laquelle structure deux courtes chaînes peptidiques se trouvent liées, et à l'extrémité de laquelle structure sont greffés par des liaisons N-glycosidique respectivement deux acides bêta-hydroxymyristique représentant environ 1,5 % en poids du composé, procédé caractérisé en ce que l'on délipide partiellement ledit composé de sorte que lui sont associés sous forme libre ou estérifiée des acides gras palmitiques représentant moins de O,5 % en poids du composé, et qu'au moins 85 % en poids, de préférence 90 % du composé, ne se présente pas en solution aqueuse sous forme d'associations du type micelle ou d'agrégats, et ses dérivés.

2. Procédé selon la revendication précédente, caractérisé en ce que la teneur en acides gras palmitiques qui lui sont liés sous forme estérifiée ne dépasse pas 0,01 % en poids du composé et la teneur en acides gras palmitiques qui lui sont associés sous forme libre ne dépasse pas O,1 % en poids du composé.

3. Procédé de préparation d'un composé selon l'une des revendications précédentes, caractérisé en ce que :
a) à partir de lysats bactériens d'une souche bactérienne gram négatif on extrait le protéoglycane membranaire brut,
b) on solubilise le protéoglycane brut de l'étape a) par hydrolyse alcaline, avec de la soude à molarité comprise entre 0,3 et 1M, de sorte que les acides gras palmitiques estérifiés sur les acides bêta-hydroxymyristique sont désestérifiés au moins en partie, et on récupère le protéoglycane soluble qui se trouve dans la solution aqueuse,
c) on isole un composé polysaccharidique et si nécessaire on élimine les protéines présentes dans la fraction isolée,
d) on extrait les acides gras libres associés audit composé polysaccharidique à l'aide d'un solvant organique approprié.

4. Procédé selon la revendication 3, caractérisé en ce que :
a) à partir de lysats bactériens d'une souche bactérienne gram négatif, on extrait le protéoglycane membranaire brut,
b) on solubilise le protéoglycane brut obtenu de l'étape a) par hydrolyse alcaline avec la soude à molarité comprise entre 0,3 et 1M, de préférence 0,5 à 0,75 M, à une température inférieure à 90°C, de préférence entre 50 et 60°C, le traitement étant poursuivi pendant au moins une heure,
c) après refroidissement, on neutralise la suspension par un acide, et si nécessaire on effectue une hydrolyse enzymatique des protéines présentes dans la solution,
d) un composé polysaccharidique est ensuite purifié par précipitation alcoolique et recueilli par exemple par centrifugation,
e) les acides gras libres sont extraits à l'aide d'un solvant organique approprié par exemple du chloroforme ou un mélange de chloroforme et de méthanol, en dispersant ledit composé polysaccharidique dans ledit solvant organique, puis en éliminant le solvant et en rinçant le précipité après élimination du solvant de préférence par le même solvant,
f) le précipité est repris dans l'eau et une solution de D 25 délipidée selon l'invention est clarifiée par centrifugation, puis le surnageant est recueilli, et enfin,
g) un composé D 25 délipidé est isolé par un procédé de fractionnement ou l'ultrafiltration.

5. Procédé selon la revendication 4, utile pour obtenir notamment un très faible taux de polymérisation notamment inférieur à 2 %, caractérisé en ce qu'après l'étape f), on effectue une nouvelle hydrolyse alcaline ; puis après refroidissement on neutralise la suspension par un acide et éventuellement on ajoute à la solution du désoxycholate de sodium dans une proportion ne dépassant pas 0,5 % ; puis on précipite le composé polysaccharidique ; le précipité est repris dans l'eau et le composé D 25 délipidé obtenu est isolé par fractionnement ou ultrafiltration.

6. Procédé de préparation d'une composition destinée à l'imagerie, au diagnostic ou à la thérapie, caractérisé en ce qu'on introduit dans un excipient approprié le composé D 25 partiellement délipidé ou un de ses dérivés obtenu selon le procédé d'une des revendications précédentes.

7. Procédé selon la revendication 6, caractérisé en ce que la composition est destinée à l'imagerie et au diagnostic des foyers infectieux, inflammatoires et tumoraux via le ciblage des macrophages.

8. Procédé selon l'une des revendications 6 et 7 caractérisé en ce que l'excipient est un excipient approprié pour que la composition soit injectable par voie i.v..

9. Procédé selon l'une des revendications 6 et 7, caractérisé en ce que l'excipient est approprié pour que la composition soit injectable par voie lymphatique, notamment par voie sous cutanée ou voie intrapleurale.

10. Procédé selon l'une des revendications 6 et 7, caractérisé en ce que la composition est administrable par voie aérosol.

11. Procédé de préparation d'une composition d'imagerie ou de diagnostic selon l'une des revendications 6 à 10 caractérisé en ce qu'on effectue le marquage du composé polysaccharidique D 25 partiellement délipidé par un élément détectable tel qu'un élément radioactif, paramagnétique ou fluorescent.

12. Procédé selon la revendication 11 caractérisé en ce que l'élément radioactif est un radionucléide détectable par scintigraphie.

13. Procédé selon la revendication 12 caractérisé en ce que le radionucléide est le technétium ^{99m}, l'iode ¹²³, ou l'indium ¹¹¹.

## Claims (Claims for the following Contracting State(s): DE, GB, FR, IT, NL, SE, CH, LI, BE, AT, LU, DK)

1. Polysaccharide compound D25 having a molecular weight of 30 kD, extracted from membrane proteoglycans of the bacterium Klebsiella pneumoniae comprising a linear polysaccharide chain composed of the repetition of a sequence containing only galactoses at the terminus of which is found a unique linkage structure containing glucose, galactose, glucosamine, heptose and mannodeoxyoctulosonic acid residues, two short peptide chains are bound to this structure and at the terminus of this structure are grafted, through N-glycosidic bonds, two beta-hydroxymyristic acids respectively, representing about 1.5% by weight of the compound, from which compound the lipid has been partially removed so that palmitic fatty acids representing less than 0.5% by weight of the compound are associated with it in free or esterified form, and so that at least 85% by weight, preferably 90% of the compound, does not exist in aqueous solution in the form of micelle type associations or aggregates, and its derivatives.

2. Compound according to the preceding claim, characterized in that the amount of palmitic fatty acids which is bound to it in esterified form does not exceed 0.01% by weight of the compound and the amount of palmitic fatty acids which is associated with it in the free form does not exceed 0.1% by weight of the compound.

3. Process for the preparation of a compound according to one of the preceding claims, characterized in that:
a) the crude membrane proteoglycan is extracted from bacterial lysates of a Gram-negative bacterial strain,
b) the crude proteoglycan of step a) is solubilized by alkaline hydrolysis, using sodium hydroxide of molarity between 0.3 and 1 M, so that the palmitic fatty acids esterified on the beta-hydroxymyristic acids are at least partially de-esterified, and the soluble proteoglycan which is present in the aqueous solution is recovered,
c) a polysaccharide compound is isolated and if necessary the proteins which are present in the isolated fraction are removed,
d) the free fatty acids associated with the said polysaccharide compound are extracted using a suitable organic solvent.

4. Process according to Claim 3, characterized in that:
a) the crude membrane proteoglycan is extracted from bacterial lysates of a Gram-negative bacterial strain,
b) the crude proteoglycan obtained in step a) is solubilized by alkaline hydrolysis, using sodium hydroxide of molarity between 0.3 and 1 M, preferably from 0.5 to 0.75 M at a temperature lower than 90°C, preferably between 50 and 60°C, the treatment being continued for at least one hour,
c) after cooling, the suspension is neutralized with an acid, and if necessary an enzymatic hydrolysis of the proteins present in the solution is carried out,
d) a polysaccharide compound is then purified by alcohol precipitation and recovered for example by centrifugation,
e) the free fatty acids are extracted using a suitable organic solvent, for example chloroform or a mixture of chloroform and methanol, by dispersing the said polysaccharide compound in the said organic solvent, then removing the solvent and rinsing the precipitate after removing the solvent preferably with the same solvent,
f) the precipitate is resuspended in water, and a solution of lipid-free D 25 according to the invention is clarified by centrifugation, then the supernatant is recovered, and finally
g) a lipid-free D 25 compound is isolated by a fractionation or ultrafiltration process.

5. Process according to Claim 4, which is useful for obtaining in particular a very low degree of polymerization, in particular lower than 2%, characterized in that after step f) a new alkaline hydrolysis is carried out, then, after cooling, the suspension is neutralized with an acid and sodium deoxycholate is optionally added to the solution in a proportion not exceeding 0.5%, then the polysaccharide compound is precipitated, and the precipitate is suspended in water, and then the lipid-free D 25 compound obtained is isolated by fractionation or ultrafiltration.

6. Composition intended for imaging, diagnosis or therapy, characterized in that it comprises the D 25 compound whose lipid has been partially removed or one of its derivatives according to one of the preceding claims.

7. Composition according to Claim 6, intended for the imaging and the diagnosis of infectious, inflammatory and tumour foci through the targeting of macrophages.

8. Composition according to one of Claims 6 and 7, characterized in that it is injectable by the iv. route.

9. Composition according to one of Claims 6 and 7, characterized in that it is injectable by the lymphatic route, in particular by the subcutaneous route or the intrapleural route.

10. Composition according to one of claims 6 and 7, characterized in that it is administrable by the aerosol route.

11. Imaging or diagnostic composition according to one of Claims 6 to 10, characterized in that the D 25 polysaccharide compounds whose lipid has been partially removed is labelled with a detectable element such as a radioactive, paramagnetic or fluorescent element.

12. Imaging and diagnostic composition according to one of Claims 6 to 10, characterized in that it comprises the polysaccharide compound according to the invention, nonlabelled, and in that the composition constitutes part of an imaging and diagnostic kit comprising a second composition containing a substance, in particular an antibody, capable of recognizing the said polysaccharide compound, the said substance itself being labelled with a detectable element such as a radioactive, paramagnetic or fluorescent element.

13. Composition according to one of Claims 11 and 12, characterized in that the radioactive element is a radionuclide which is detectable by scintigraphy.

14. Composition according to Claim 13, characterized in that the radionuclide is ^{99m}technetium, ¹²³iodine or ¹¹¹indium.

15. Use of the compound according to one of Claims 1 to 5 for therapeutic purposes as transporting agent for targeted radiotherapy, for the transport of cytotoxic compounds or for the transport of immunogenic substances and/or immunomodulating agents.

16. Use of the compound according to one of Claims 1 to 5 as a medicament.

## Claims (Claims for the following Contracting State(s): ES, GR)

1. Process for the preparation of a polysaccharide compound D25 having a molecular weight of 30 kD, extracted from membrane proteoglycans of the bacterium Klebsiella pneumoniae comprising a linear polysaccharide chain composed of the repetition of a sequence containing only galactoses at the terminus of which is found a unique linkage structure containing glucose, galactose, glucosamine, heptose and mannodeoxyoctulosonic acid residues, two short peptide chains are bound to this structure and at the terminus of this structure are grafted, through N-glycosidic bonds, two beta-hydroxymyristic acids respectively, representing about 1.5% by weight of the compound, the process being characterized in that the lipid of the said compound is partially removed so that palmitic fatty acids representing less than 0.5% by weight of the compound are associated with it in free or esterified form, and so that at least 85% by weight, preferably 90% of the compound, does not exist in aqueous solution in the form of micelle type associations or aggregates, and its derivatives.

2. Process according to the preceding claim, characterized in that the amount of palmitic fatty acids which is bound to it in esterified form does not exceed 0.01% by weight of the compound and the amount of palmitic fatty acids which is associated with it in the free form does not exceed 0.1% by weight of the compound.

3. Process for the preparation of a compound according to one of the preceding claims, characterized in that:
a) the crude membrane proteoglycan is extracted from bacterial lysates of a Gram-negative bacterial strain,
b) the crude proteoglycan of step a) is solubilized by alkaline hydrolysis, using sodium hydroxide of molarity between 0.3 and 1 M, so that the palmitic fatty acids esterified on the beta-hydroxymyristic acids are at least partially de-esterified, and the soluble proteoglycan which is present in the aqueous solution is recovered,
c) a polysaccharide compound is isolated and if necessary the proteins which are present in the isolated fraction are removed,
d) the free fatty acids associated with the said polysaccharide compound are extracted using a suitable organic solvent.

4. Process according to Claim 3, characterized in that:
a) the crude membrane proteoglycan is extracted from bacterial lysates of a Gram-negative bacterial strain,
b) the crude proteoglycan obtained in step a) is solubilized by alkaline hydrolysis, using sodium hydroxide of molarity between 0.3 and 1 M, preferably from 0.5 to 0.75 M at a temperature lower than 90°C, preferably between 50 and 60°C, the treatment being continued for at least one hour,
c) after cooling, the suspension is neutralized with an acid, and if necessary an enzymatic hydrolysis of the proteins present in the solution is carried out,
d) a polysaccharide compound is then purified by alcohol precipitation and recovered for example by centrifugation,
e) the free fatty acids are extracted using a suitable organic solvent, for example chloroform or a mixture of chloroform and methanol, by dispersing the said polysaccharide compound in the said organic solvent, then removing the solvent and rinsing the precipitate after removing the solvent preferably with the same solvent,
f) the precipitate is resuspended in water, and a solution of lipid-free D 25 according to the invention is clarified by centrifugation, then the supernatant is recovered, and finally
g) a lipid-free D 25 compound is isolated by a fractionation or ultrafiltration process.

5. Process according to claim 4, which is useful for obtaining in particular a very low degree of polymerization, in particular lower than 2%, characterized in that after step f) a new alkaline hydrolysis is carried out, then, after cooling, the suspension is neutralized with an acid and sodium deoxycholate is optionally added to the solution in a proportion not exceeding 0.5%, then the polysaccharide compound is precipitated, and the precipitate is suspended in water, and then the lipid-free D 25 compound obtained is isolated by fractionation or ultrafiltration.

6. Process for the preparation of a composition intended for imaging, diagnosis or therapy, characterized in that the D 25 compound whose lipid has been partially removed or one of its derivatives obtained according to the process of one of the preceding claims is introduced into a suitable excipient.

7. Process according to Claim 6, characterized in that the composition is intended for the imaging and the diagnosis of infectious, inflammatory and tumour foci through the targeting of macrophages.

8. Process according to one of Claims 6 and 7, characterized in that the excipient is a suitable excipient for the composition to be injectable by the i.v. route.

9. Process according to one of Claims 6 and 7, characterized in that the excipient is suitable for the composition to be injectable by the lymphatic route, in particular by the subcutaneous route or the intrapleural route.

10. Process according to one of Claims 6 and 7, characterized in that the composition is administrable by the aerosol route.

11. Process for the preparation of an imaging or diagnostic composition according to one of Claims 6 to 10, characterized in that the D 25 polysaccharide compounds whose lipid has been partially removed is labelled with a detectable element such as a radioactive, paramagnetic or fluorescent element.

12. Process according to Claim 11, characterized in that the radioactive element is a radionuclide which is detectable by scintigraphy.

13. Process according to Claim 12, characterized in that the radionuclide is ^{99m}technetium, ¹²³iodine or ¹¹¹indium.

## Patentansprüche (Patentansprüche für folgende(n) Vertragsstaat(en): DE, GB, FR, IT, NL, SE, CH, LI, BE, AT, LU, DK)

1. Polysaccharid-Verbindung D 25 mit einem Molekulargewicht von 30 kD, die aus Membran-Proteoglycanen des Bacteriums Klebsiella pneumoniae extrahiert worden ist, die umfaßt eine lineare Polysaccharid-Kette, die besteht aus der Wiederholung einer Sequenz, die nur Galactosen enthält und an deren Ende sich eine einzelne Bindungsstruktur befindet, die Reste von Glucosen, Galactose, Glucosamin, Heptose und Mannodesoxyoctuloson-Säure enthält, wobei an diese Struktur zwei kurze Peptidketten gebunden sind und auf das Ende dieser Struktur über N-glycosidische Bindungen jeweils zwei β-Hydroxymyristinsäuren aufgepfropft sind, die etwa 1,5 Gew.-% der Verbindung ausmachen, wobei die Verbindung teilweise so delipidiert ist, daß ihr in freier Form oder in veresterter Form Palmitinfettsäuren assoziiert sind, die weniger als 0,5 Gew.-% der Verbindung ausmachen, und daß mindestens 85 Gew.-%, vorzugsweise 90 Gew.-%, der Verbindung nicht in wäßriger Lösung in Form von Assoziationen vom Micellen- oder Aggregat-Typ vorliegen, und ihre Derivate.

2. Verbindung nach dem vorhergehenden Anspruch, dadurch gekennzeichnet, daß der Gehalt an Palmitin-Fettsäuren, die in veresterter Form an sie gebunden sind, 0,01 Gew.-% der Verbindung nicht übersteigt, und daß der Gehalt an Palmitin-Fettsäuren, die mit ihr in freier Form assoziiert sind, 0,1 Gew.-% der Verbindung nicht übersteigt.

3. Verfahren zur Herstellung einer Verbindung nach einem der vorhergehenden Ansprüche, dadurch gekennzeichnet, daß man
a) ausgehend von Bakterien-Lysaten eines Gram-negativen Bakterien-Stammes das rohe Membran-Proteoglycan extrahiert,
b) das rohe Proteoglycan der Stufe (a) durch alkalische Hydrolyse mit 0,3 bis 1 M Natriumhydroxid so solubilisiert, daß die veresterten Palmitin-Fettsäuren auf den β-Hydroxymyristinsäuren mindestens zum Teil entestert werden und daß man das lösliche Proteoglycan, das sich in der wäßrigen Lösung befindet, gewinnt (abtrennt),
c) eine Polysaccharid-Verbindung isoliert und erforderlichenfalls die in der isolierten Fraktion vorhandenen Proteine eliminiert und
d) die mit der Polysaccharid-Verbindung assoziierten freien Fettsäuren mit Hilfe eines geeigneten organischen Lösungsmittels extrahiert.

4. Verfahren nach Anspruch 3, dadurch gekennzeichnet, daß man
a) ausgehend von Bakterien-Lysaten eines Gram-negativen Bakterien-Stammes das rohe Membran-Proteoglycan extrahiert,
b) das in der Stufe (a) erhaltene rohe Proteoglycan durch alkalische Hydrolyse mit 0,3 bis 1 M, vorzugsweise 0,5 bis 0,75 M Natriumhydroxid bei einer Temperatur unterhalb 90°C, vorzugsweise zwischen 50 und 60°C, solubilisiert, wobei die Behandlung mindestens 1 h lang fortgesetzt wird,
c) nach der Abkühlung die Suspension mit einer Säure neutralisiert und erforderlichenfalls eine enzymatische Hydrolyse der in der Lösung vorhandenen Proteine durchfuhrt,
d) eine Polysaccharid-Verbindung anschließend durch alkoholische Fällung reinigt und beispielsweise durch Zentrifugieren gewinnt (abtrennt),
e) die freien Fettsäuren mit Hilfe eines geeigneten organischen Lösungsmittels, beispielsweise mit Chloroform oder einem Gemisch aus Chloroform und Methanol, extrahiert, indem man die genannte Polysaccharid-Verbindung in dem genannten organischen Lösungsmittel dispergiert, dann das Lösungsmittel eliminiert und den Niederschlag nach der Eliminierung des Lösungsmittels vorzugsweise mit dem gleichen Lösungsmittel spült,
f) den Niederschlag in Wasser wieder aufnimmt und eine Lösung des erfindungsgemäßen delipidierten D 25 durch Zentrifugieren klärt, dann die überstehende Flüssigkeit abtrennt und schließlich
g) eine delipidierte Verbindung D 25 durch ein Fraktionier- oder Ultrafiltrationsverfahren isoliert.

5. Verfahren nach Anspruch 4, das anwendbar (nützlich ist) für die Erzielung insbesondere eines sehr geringen Polymerisationsgrades, insbesondere von weniger als 2 %, dadurch gekennzeichnet, daß man nach der Stufe (f) eine neue alkalische Hydrolyse durchführt; dann nach der Abkühlung die Suspension mit einer Säure neutralisiert und gegebenenfalls zu der Lösung Natriumdesoxycholat in einem Mengenanteil, der 0,5 % nicht übersteigt, zugibt; danach die Polysaccharid-Verbindung ausfällt; den Niederschlag in Wasser wieder aufnimmt und die erhaltene, delipidierte Verbindung D 25 durch Fraktionierung oder Ultrafiltration isoliert.

6. Zusammensetzung für die bildliche Darstellung, für die Diagnose oder die Therapie, dadurch gekennzeichnet, daß sie die teilweise delipidierte Verbindung D 25 oder eines ihrer Derivate nach einem der vorhergehenden Ansprüche enthält.

7. Zusammensetzung nach Anspruch 6 für die bildliche Darstellung (Bilderzeugung) und für die Diagnose von Infektions-, Entzündungs- und Tumorherden über die Ansteuerung (das Ansprechen) von Makrophagen.

8. Zusammensetzung nach einem der Ansprüche 6 und 7, dadurch gekennzeichnet, daß sie auf intravenösem Wege injizierbar ist.

9. Zusammensetzung nach einem der Ansprüche 6 und 7, dadurch gekennzeichnet, daß sie auf lymphatischem Wege, insbesondere auf subkutanem Wege oder intrapleuralem Wege, injizierbar ist.

10. Zusammensetzung nach einem der Ansprüche 6 und 7, dadurch gekennzeichnet, daß sie in Form eines Aerosols verabreichbar ist.

11. Zusammensetzung für die bildliche Darstellung oder Diagnose nach einem der Ansprüche 6 bis 10, dadurch gekennzeichnet, daß die partiell delipidierte Polysaccharid-Verbindung D 25 mit einem nachweisbaren Element, beispielsweise einem radioaktiven, paramagnetischen oder fluoreszierenden Element, markiert ist.

12. Zusammensetzung für die bildliche Darstellung und Diagnose nach einem der Ansprüche 6 bis 10, dadurch gekennzeichnet, daß sie die nicht-markierte erfindungsgemäße Polysaccharid-Verbindung enthält und daß die Zusammensetzung Teil eines Kits für die bildliche Darstellung (Bilderzeugung) und die Diagnose ist, der umfaßt eine zweite Zusammensetzung, enthaltend eine Substanz, welche die genannte Polysaccharid-Verbindung erkennen kann, insbesondere einen Antikörper, wobei die genannte Substanz selbst markiert ist durch ein nachweisbares Element, beispielsweise ein radioaktives, paramagnetisches oder fluoreszierendes Element.

13. Zusammensetzung nach einem der Ansprüche 11 und 12, dadurch gekennzeichnet, daß das radioaktive Element ein durch Szintigraphie nachweisbares Radionuclid ist.

14. Zusammensetzung nach Anspruch 13, dadurch gekennzeichnet, daß das Radionuclid Technetium^{99m}, Jod¹²³ oder Indium¹¹¹ ist.

15. Verwendung der Verbindung nach einem der Ansprüche 1 bis 5 zu therapeutischen Zwecken als Vektorisierungsmittel für die gezielte Radiotherapie, für die Vektorisierung von zytotoxischen Substanzen oder auch für die Vektorisierung von immunogenen Substanzen und/oder Immunmodulator-Agentien.

16. Verwendung der Verbindung nach einem der Ansprüche 1 bis 5 als Arzneimittel.

## Patentansprüche (Patentansprüche für folgende(n) Vertragsstaat(en): ES, GR)

1. Verfahren zur Herstellung einer Polysaccharid-Verbindung D 25 mit einem Molekulargewicht von 30 kD, die aus Membran-Proteoglycanen des Bacteriums Klebsiella pneumoniae extrahiert worden ist, die umfaßt eine lineare Polysaccharid-Kette, die besteht aus der Wiederholung einer Sequenz, die nur Galactosen enthält und an deren Ende sich eine einzelne Bindungsstruktur befindet, die Reste von Glucosen, Galactose, Glucosamin, Heptose und Mannodesoxyoctuloson-Säure enthält, wobei auf der genannten Struktur zwei kurze Peptidketten miteinander verbunden sind und auf das Ende der Struktur durch N-glycosidische Bindungen jeweils zwei β-Hydroxymyristinsäuren aufgepfropft sind, die etwa 1,5 Gew.-% der Verbindung darstellen, dadurch gekennzeichnet, daß man die genannte Verbindung teilweise so delipidiert, daß ihr Palmitin-Fettsäuren in der freien oder veresterten Form assoziiert sind, die weniger als 0,5 Gew.-% der Verbindung ausmachen, und daß mindestens 85 Gew.-%, vorzugsweise 90 Gew.-%, der Verbindung nicht in wäßriger Lösung vorliegen in Form von Assoziationen vom Micellen- oder Aggregat-Typ, und ihrer Derivate.

2. Verfahren nach dem vorhergehenden Anspruch, dadurch gekennzeichnet, daß der Gehalt an Palmitin-Fettsäuren, mit denen sie in veresterter Form verbunden ist, 0,01 Gew.-% der Verbindung nicht übersteigt und daß der Gehalt an Palmitin-Fettsäuren, mit denen sie in freier Form assoziiert ist, 0,1 Gew.-% der Verbindung nicht übersteigt.

3. Verfahren zur Herstellung einer Verbindung nach einem der vorhergehenden Ansprüche, dadurch gekennzeichnet, daß man
a) ausgehend von Bakterien-Lysaten eines Gram-negativen Bakterien-Stammes das rohe Membran-Proteoglycan extrahiert,
b) das rohe Proteoglycan der Stufe (a) durch alkalische Hydrolyse mit 0,3 bis 1 M Natriumhydroxid so solubilisiert, daß die veresterten Palmitin-Fettsäuren auf den β-Hydroxymyristinsäuren mindestens zum Teil entestert werden und daß man das lösliche Proteoglycan, das sich in der wäßrigen Lösung befindet, gewinnt (abtrennt),
c) eine Polysaccharid-Verbindung isoliert und erforderlichenfalls die in der isolierten Fraktion vorhandenen Proteine eliminiert und
d) die mit der Polysaccharid-Verbindung assoziierten freien Fettsäuren mit Hilfe eines geeigneten organischen Lösungsmittels extrahiert.

4. Verfahren nach Anspruch 3, dadurch gekennzeichnet, daß man
a) ausgehend von Bakterien-Lysaten eines Gram-negativen Bakterien-Stammes das rohe Membran-Proteoglycan extrahiert,
b) das in der Stufe (a) erhaltene rohe Proteoglycan durch alkalische Hydrolyse mit 0,3 bis 1 M, vorzugsweise 0,5 bis 0,75 M Natriumhydroxid bei einer Temperatur unterhalb 90°C, vorzugsweise zwischen 50 und 60°C, solubilisiert, wobei die Behandlung mindestens 1 h lang fortgesetzt wird,
c) nach der Abkühlung die Suspension mit einer Säure neutralisiert und erforderlichenfalls eine enzymatische Hydrolyse der in der Lösung vorhandenen Proteine durchführt,
d) eine Polysaccharid-Verbindung anschließend durch alkoholische Fällung reinigt und beispielsweise durch Zentrifugieren gewinnt (abtrennt),
e) die freien Fettsäuren mit Hilfe eines geeigneten organischen Lösungsmittels, beispielsweise mit Chloroform oder einem Gemisch aus Chloroform und Methanol, extrahiert, indem man die genannte Polysaccharid-Verbindung in dem genannten organischen Lösungsmittel dispergiert, dann das Lösungsmittel eliminiert und den Niederschlag nach der Eliminierung des Lösungsmittels vorzugsweise mit dem gleichen Lösungsmittel spült,
f) den Niederschlag in Wasser wieder aufnimmt und eine delipidierte Lösung des erfindungsgemäßen D 25 durch Zentrifugieren klärt, dann die überstehende Flüssigkeit abtrennt und schließlich
g) eine delipidierte Verbindung D 25 durch ein Fraktionier- oder Ultrafiltrationsverfahren isoliert.

5. Verfahren nach Anspruch 4, das anwendbar (nützlich) ist für die Erzielung insbesondere eines sehr geringen Polymerisationsgrades, insbesondere von weniger als 2 %, dadurch gekennzeichnet, daß man nach der Stufe (f) eine neue alkalische Hydrolyse durchführt; dann nach der Abkühlung die Suspension mit einer Säure neutralisiert und gegebenenfalls zu der Lösung Natriumdesoxycholat in einem Mengenanteil, der 0,5 % nicht übersteigt, zugibt; danach die Polysaccharid-Verbindung ausfällt; den Niederschlag in Wasser wieder aufnimmt und die erhaltene, delipidierte Verbindung D 25 durch Fraktionierung oder Ultrafiltration isoliert.

6. Verfahren zur Herstellung einer Zusammensetzung für die bildliche Darstellung, für die Diagnose oder für die Therapie, dadurch gekennzeichnet, daß man die partiell delipidierte Verbindung D 25 oder eines ihrer Derivate, die (das) nach dem Verfahren nach einem der vorhergehenden Ansprüche erhalten wurde, in einen geeigneten Exzipienten einführt.

7. Verfahren nach Anspruch 6, dadurch gekennzeichnet, daß die Zusammensetzung bestimmt ist für die bildliche Darstellung (Bilderzeugung) und für die Diagnose von Infektions-, Entzündungs- und Tumorherden durch Ansteuern (Ansprechen) von Makrophagen.

8. Verfahren nach einem der Ansprüche 6 und 7, dadurch gekennzeichnet, daß der Exzipient ein geeigneter Exzipient ist, so daß die Zusammensetzung auf intravenösem Wege injizierbar ist.

9. Verfahren nach einem der Ansprüche 6 und 7, dadurch gekennzeichnet, daß der Exzipient ein geeigneter Exzipient ist, so daß die Zusammensetzung auf lymphatischem Wege, insbesondere auf subkutanem Wege oder intrapleuralem Wege, injizierbar ist.

10. Verfahren nach einem der Ansprüche 6 und 7, dadurch gekennzeichnet, daß die Zusammensetzung in Form eines Aerosols verabreichbar ist.

11. Verfahren zur Herstellung einer Zusammensetzung für die bildliche Darstellung oder die Diagnose nach einem der Ansprüche 6 bis 10, dadurch gekennzeichnet, daß man die Markierung der teilweise delipidierten Polysaccharid-Verbindung D 25 mit einem nachweisbaren Element, beispielsweise einem radioaktiven, paramagnetischen oder fluoreszierenden Element durchführt.

12. Verfahren nach Anspruch 11, dadurch gekennzeichnet, daß das radioaktive Element ein durch Szintigraphie nachweisbares Radionuclid ist.

13. Verfahren nach Anspruch 12, dadurch gekennzeichnet, daß das Radionuclid Technetium^{99m}, Jod¹²³ oder Indium¹¹¹ ist.
